(19) **Europäisches Patentamt / European Patent Office / Office européen des brevets**

(11) **EP 4 582 470 A1**

(12) # EUROPEAN PATENT APPLICATION
published in accordance with Art. 153(4) EPC

(43) Date of publication:
**09.07.2025 Bulletin 2025/28**

(21) Application number: **23860282.5**

(22) Date of filing: **28.08.2023**

(51) International Patent Classification (IPC):
**C08J 9/04** (2006.01)    **C12N 1/00** (2006.01)

(52) Cooperative Patent Classification (CPC):
**C08J 9/04; C12N 1/00; Y02W 10/10**

(86) International application number:
**PCT/JP2023/030990**

(87) International publication number:
**WO 2024/048520 (07.03.2024 Gazette 2024/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.08.2022  JP 2022138593**

(71) Applicant: **JSP Corporation
Tokyo 100-0005 (JP)**

(72) Inventors:
- **IWAYAMA, Yoshihiro
  Kanuma-shi, Tochigi 322-0014 (JP)**
- **SUNAGA, Keisuke
  Kanuma-shi, Tochigi 322-0014 (JP)**

(74) Representative: **Dehns
10 Old Bailey
London EC4M 7NG (GB)**

(54) **MICROBIAL CARRIER FOR FOOD WASTE TREATMENT**

(57)    A microbial carrier for food waste treatment, wherein the base resin constituting the microbial carrier comprises thermoplastic starch and/or aliphatic polyester, the microbial carrier has a biodegradability of 60% or higher in a biodegradability test based on JIS K6953, and the microbial carrier has a columnar shape.

**FIG.7**

EP 4 582 470 A1

## Description

Technical Field

This invention relates to a microbial carrier for food waste treatment that carries microorganisms for processing food waste.

Background of Art

Most food waste is treated through incineration at incineration plants or by landfill. Incineration generates large amounts of exhaust heat and carbon dioxide, raising concerns about its environmental impact. Additionally, securing landfill sites poses significant challenges.

Thus, various technologies have been proposed to treat food waste without relying on incineration or landfilling. For instance, technologies that use microorganisms to biodegrade food waste have minimal environmental impact and eliminate the need for landfill sites. Food waste is decomposed into water and carbon dioxide by microorganisms. By stirring the food waste with a microbial carrier in a waste treatment apparatus, the food waste is decomposed.

For example, Patent Literature 1 and Patent Literature 2 disclose technologies related to carriers for microorganisms. The carrier in Patent Literature 1 contains thermoplastic resin, while the carrier in Patent Literature 2 includes polyolefin and polystyrene.

Citation List

Patent Literature

Patent Literature 1: JP 2003-55562 A
Patent Literature 2: JP 6869307 B

Summary of Invention

Technical Problem

Here, the carrier, after being used for a certain period, experiences a decrease in biodegradability or the attachment of odors. For this reason, the carrier is replaced approximately every two years. As a result, used carriers from Patent Literature 1 and Patent Literature 2 become a large amount of waste, necessitating incineration or landfill disposal of the carriers, as mentioned above. Therefore, conventional carriers posed issues such as environmental impact and securing landfill sites.

In consideration of the above circumstances, this invention aims to provide a microbial carrier for food waste treatment that eliminates the need for incineration or landfill disposal of the carrier, or significantly reduces the amount of discarded carriers, thereby minimizing environmental impact.

Means for Solving the Problem

[1] A microbial carrier for food waste treatment,
wherein the base resin constituting the microbial carrier comprises thermoplastic starch and/or aliphatic polyester, the microbial carrier has a biodegradability of 60% or higher in a biodegradability test based on JIS K6953, and the microbial carrier has a columnar shape.

[2] The microbial carrier for food waste treatment according to [1],
wherein the arithmetic average height (Sa) of surface irregularities on the surface of the microbial carrier is 2.4 $\mu$m or more.

[3] The microbial carrier for food waste treatment according to [1] or [2],
wherein the apparent density of the microbial carrier is 400 kg/m$^3$ or more and 800 kg/m$^3$ or less.

[4] The microbial carrier for food waste treatment according to any one of [1] to [3],
wherein the cross-sectional shape of the microbial carrier, when cut perpendicularly to the height direction, is polygonal.

[5] The microbial carrier for food waste treatment according to any one of [1] to [4],
wherein the plant-based content of the thermoplastic starch or aliphatic polyester, as determined based on ASTM D6866, is 40% or more.

Effect of the Invention

The microbial carrier for food waste treatment of this invention is biodegradable. Therefore, it eliminates the need for incineration or landfill disposal of the carrier, or significantly reduces the amount of discarded carriers, thereby reducing the environmental impact.

Brief Description of the Drawings

[Fig. 1] A perspective view of a carrier according to an example of the present invention.
[Fig. 2] A perspective view of a carrier according to an example of the present invention.
[Fig. 3] A perspective view of a carrier according to an example of the present invention.
[Fig. 4] A perspective view of a carrier according to an example of the present invention.
[Fig. 5] A perspective view of a carrier according to an example of the present invention.
[Fig. 6] A perspective view of a carrier according to an example of the present invention.
[Fig. 7] A perspective view of a carrier according to an example of the present invention.
[Fig. 8] An electron microscope image (magnified 10,000 times) of the surface of the side portion of a carrier according to an example of the present invention.

Modes for Carrying Out the Invention

The microbial carrier for food waste treatment according to the present invention (hereinafter simply referred to as the "carrier") is a structure designed to carry microorganisms for processing food waste. The microbial carrier for food waste treatment according to the present invention encompasses cases where microorganisms are carried on the carrier and cases where they are not. Food waste refers to residues generated during the cooking process, unsold items arising during the distribution process, and leftovers produced at the consumption stage.

In the following description, preferred numerical ranges of the present invention may be provided as appropriate. In such cases, the preferred range, more preferred range, and particularly preferred range concerning the upper and lower limits of the numerical range can be determined from all combinations of the upper and lower limits. Additionally, the numerical range "A to B" is synonymous with "A or more and B or less," and is used to include the endpoints (A, B) of the range. Furthermore, "A and/or B" includes the concepts of "A and B" as well as "either A or B."

The carrier of the present invention pulverizes food waste. In a treatment apparatus, the food waste is pulverized by the stirring of the carrier and decomposed into water and carbon dioxide by the microorganisms carried on the carrier. As described above, the carrier has the functions of carrying microorganisms and pulverizing food waste.

Specifically, the carrier of the present invention has a biodegradability of 60% or higher, preferably 80% or higher, and more preferably 90% or higher in a biodegradability test based on JIS K6953. In other words, the carrier of the present invention is capable of being biodegraded by microorganisms. Therefore, the carrier of the present invention eliminates the need for incineration or landfill disposal of the used carrier, or significantly reduces the amount of carrier waste.

The biodegradability test based on JIS K6953 involves placing the test sample (the carrier in the present invention) into an aerobic compost (a wellventilated compost) and calculating the biodegradability from the amount of carbon dioxide generated during decomposition by microorganisms.

The base resin of the carrier in the present invention includes thermoplastic starch and/or aliphatic polyester. That is, it is preferable that the base resin includes thermoplastic starch and aliphatic polyester, thermoplastic starch, or aliphatic polyester. Here, the base resin refers to a resin that accounts for 50% or more by weight of the total 100% weight of the resin constituting the carrier. Preferably, it accounts for 70% or more by weight, and more preferably 90% or more, with the resin constituting the carrier being made up of the base resin.

The content of thermoplastic starch and/or aliphatic polyester in the base resin is, for example, 50% by weight or more, preferably 70% by weight or more, and more preferably 90% by weight or more. It should be noted that the content of thermoplastic starch and/or aliphatic polyester in the base resin refers to the total amount when both thermoplastic starch and aliphatic polyester are included in the base resin.

Thermoplastic starch is modified starch that has been rendered thermoplastic using starch or starch derivatives. Examples of thermoplastic starch include starch in which some of the three hydroxyl groups in the glucose units, which are the structural units of starch molecular chains, have been substituted with hydrophobic groups through etherification or esterification reactions, and to which plasticizers such as glycerin or ethylene glycol have been added.

When the base resin consists of thermoplastic starch without containing aliphatic polyester, the content of thermoplastic starch in the base resin is, for example, 50% by weight or more, preferably 70% by weight or more, and more preferably 90% by weight or more.

Aliphatic polyester primarily contains aliphatic ester as the main component in its main chain. The content of aliphatic ester in the main chain is at least 60 mol%, preferably 80 to 100 mol%, and more preferably 90 to 100 mol%. Aliphatic polyester includes hydroxy acid polycondensates, ring-opening polymers of lactones, and polycondensates of polyhydric alcohol components and polycarboxylic acid components. Examples of hydroxy acid polycondensates include polylactic acid and polyhydroxybutyrate. Examples of ring-opening polymers of lactones include polycaprolactone and polypropiolactone. Examples of polycondensates of polyhydric alcohol components and polycarboxylic acid components include polybutylene succinate (PBS), polybutylene adipate (PBAT), and polyethylene succinate. Among these, polylactic acid and polybutylene succinate are preferred from the perspective of carrier moldability.

When the base resin consists of aliphatic polyester without containing thermoplastic starch, the content of aliphatic polyester in the base resin is, for example, 50% by weight or more, preferably 70% by weight or more, and more preferably 90% by weight or more.

The thermoplastic starch and aliphatic polyester used as the base resin preferably have a biodegradability of 60% or higher based on JIS K6953, more preferably 80% or higher, and even more preferably 90% or higher. The upper limit of biodegradability is 100%. Additionally, it is considered that the biodegradability of carriers made using the above-mentioned thermoplastic starch and aliphatic polyester as the base resin will have values similar to the biodegradability of the thermoplastic starch and aliphatic polyester themselves.

The plant-based content of at least one of thermoplastic starch and aliphatic polyester is preferably 20% or higher, more preferably 50% or higher, and even more preferably 70% or higher. By ensuring that the plant-based content of thermoplastic starch and aliphatic polyester falls within the above range, the carbon-neutral effect can be enhanced, contributing to the mitigation of global warming. The plant-based content can be measured using ASTM D6866.

Additionally, it is preferable that at least one of the thermoplastic starch and aliphatic polyester has a marine degradability of 90% or higher. By using such base resins, even if the carrier is damaged during waste processing and flows into rivers or oceans along with wastewater, it can decompose quickly. The marine degradability can be measured using ASTM D6691.

The base resin of the carrier according to the present invention may contain resins other than thermoplastic starch and aliphatic polyester, provided that the biodegradability of the carrier remains at 60% or higher. Such other resins may include, for example, polypropylene resins, polyester resins, polyolefin resins, polyphenylene ether resins, elastomers, and other polymers. However, the content of other resins in the base resin is preferably 10% by weight or less, more preferably 5% by weight or less, even more preferably 3% by weight or less, and most preferably 0% (i.e., the base resin contains only thermoplastic starch and/or aliphatic polyester).

The carrier may contain various other components, provided that the biodegradability of the carrier remains at 60% or higher. Such other components may include various additives, for example, blowing agents (physical blowing agents and chemical blowing agents), nucleating agents, plasticizers, pigments, dyes, coloring agents, heat stabilizers, fillers, and other additives.

The structural features of the carrier are as follows.

[Shape]

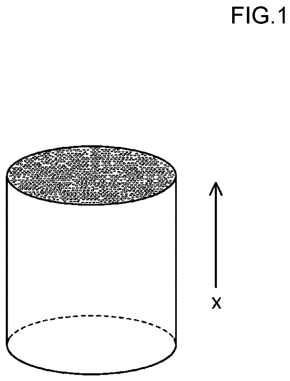

Figures 1 through 7 are perspective views of the carrier. As illustrated in Figures 1 through 7, the carrier of the present invention is columnar. Preferably, the carrier is a structure that includes a bottom surface, a top surface, and a side surface located between the bottom and top surfaces.

The cross-sectional area of the carrier (i.e., the area of the bottom and top surfaces) is, for example, 0.5 to 1.4 $cm^2$. The height of the carrier is 0.8 to 1.3 cm. The cross-sectional area of the carrier refers to the area of the cross-section when the carrier is cut along a plane perpendicular to its height direction x (axial direction). The height of the carrier refers to, for example, the distance between the bottom surface and the top surface of the columnar body shown in Figure 1.

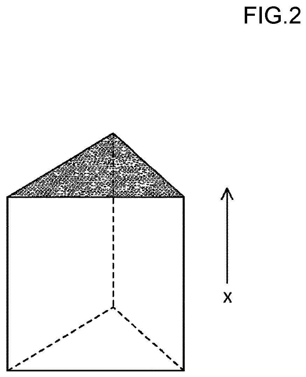

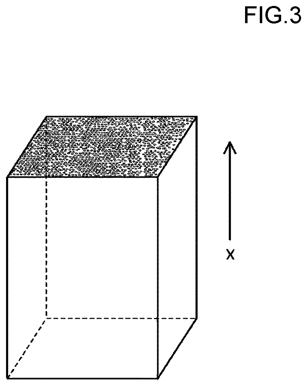

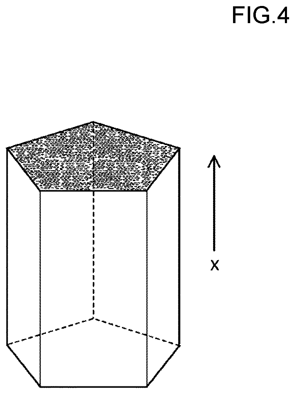

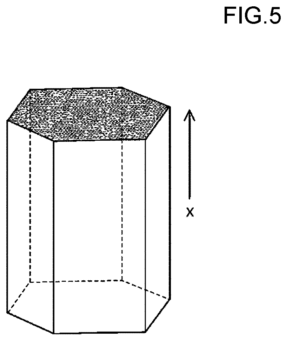

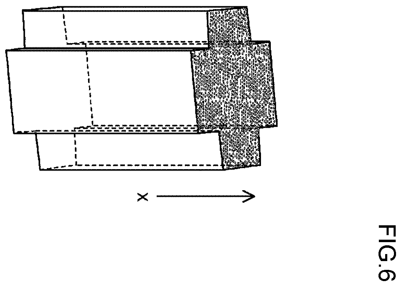

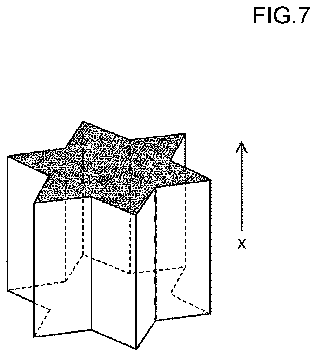

Figure 1 depicts a carrier with a circular cross-sectional shape. On the other hand, Figures 2 through 7 illustrate carriers with polygonal cross-sectional shapes. Specifically, the cross-sectional shape in Figure 2 is triangular, in Figure 3 is quadrilateral, in Figure 4 is pentagonal, in Figure 5 is hexagonal, in Figure 6 is a cross shape (formed by two rectangles intersecting orthogonally at their centroids), and in Figure 7 is star-shaped. However, the cross-sectional shape of the carrier is not limited to these examples. The term "cross-sectional shape" refers to the shape of the cross-section when the carrier is cut along a plane perpendicular to its height direction x.

When the volume is the same, a carrier with a polygonal cross-sectional shape has an increased surface area compared to a carrier with a circular cross-sectional shape, making it easier for microorganisms to adhere. Additionally, the corners improve the crushing capability, which is expected to enhance the decomposition efficiency of food waste. Therefore, a polygonal cross-sectional shape is preferred for the carrier, with a star shape being particularly preferable among polygonal shapes. However, carriers with a circular cross-sectional shape are also included within the scope of the present invention.

[Average Particle Size]

The average particle size of the carrier is preferably 5 to 20 mm, more preferably 6 to 15 mm, and even more preferably 7 to 13 mm. If the average particle size falls within this range, the carrier exhibits excellent handling properties. The average particle size can be determined by measuring the average maximum height of the columnar carrier using a caliper or similar tool. Additionally, the volume of each carrier is preferably 0.2 to 1 cm$^3$, more preferably 0.3 to 0.8 cm$^3$. If the volume of each carrier falls within this range, the carrier demonstrates excellent decomposition performance. The volume of each carrier can be calculated based on the apparent density and the weight of the individual carrier, which are measured separately.

Additionally, the ratio of the average particle size of the carrier to the maximum length of the cross-section (L/d) is preferably 1 to 2, and more preferably 1.0 to 1.6. When the (L/d) ratio falls within this range, the carrier exhibits excellent flowability within the waste treatment apparatus.

[Arithmetic Average Height (Sa)]

The arithmetic average height (Sa) of surface irregularities on the carrier's surface is preferably 2.4 to 5 $\mu$m, more preferably 2.5 to 4.5 $\mu$m, even more preferably 3.0 to 4.2 $\mu$m, and most preferably 3.2 to 4.0 $\mu$m. The arithmetic average height is an index that indicates the mean height difference from the average plane. When the Sa falls within the above range, the surface irregularities are believed to provide resistance upon contact with food waste, resulting in enhanced crushing power. Such a structure forms an uneven surface across the entire carrier, which further improves its ability to crush food waste. Specifically, the numerical range of the Sa can be defined by selecting a lower limit from the group consisting of 2.4 $\mu$m, 2.5 $\mu$m, 3.0 $\mu$m, and 3.2 $\mu$m, and an upper limit from the group consisting of 5 $\mu$m, 4.5 $\mu$m, 4.2 $\mu$m, and 4.0 $\mu$m.

In the present invention, the arithmetic average height of surface irregularities is determined by measuring the surface of the columnar carrier using any known method. For example, the arithmetic average height can be identified using analysis software from a 3D image of the carrier's side surface captured by a laser microscope (microscope). The 3D image used is corrected for flatness by applying an L-filter, which removes long-wavelength components such as waviness or shape elements.

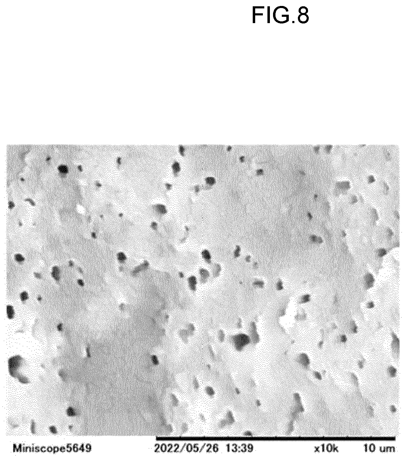

The side surface of the columnar carrier preferably has pores with diameters of approximately 0.3 to 3 $\mu$m, as shown in Figure 8. When the carrier is a porous body with such pores, it demonstrates particularly excellent microorganism adsorption properties. From this perspective, the maximum diameter of the pore is preferably 0.3 to 3 $\mu$m and more preferably 0.5 to 2 $\mu$m. The maximum diameter of the pore on the surface of the carrier refers to the absolute maximum length. For example, the numerical range of the maximum diameter of the pore can be defined by selecting a lower limit from the group consisting of 0.3 $\mu$m and 0.5 $\mu$m, and an upper limit from the group consisting of 3 $\mu$m and 2 $\mu$m.

In the electron microscope images of the side surface of the columnar carrier, it is preferable that 5 to 80 pores with a maximum diameter of 0.3 to 3 $\mu$m are formed within an area of 100 $\mu$m$^2$. The number of pores is more preferably 10 to 40 pores per 100 $\mu$m$^2$. Such pores can be formed, for example, by using a base resin blended with thermoplastic starch. Specifically, the number of pores can be defined within a range where the lower limit is selected from the group consisting of 5, 10, and 30, and the upper limit is selected from the group consisting of 80, 60, and 40.

[Apparent Density]

The apparent density of the carrier is preferably 300 to 950 kg/m$^3$, more preferably 350 to 900 kg/m$^3$, even more preferably 400 to 800 kg/m$^3$, and most preferably 450 to 750 kg/m$^3$. Within this range, the carrier has an appropriate weight and exhibits excellent crushing performance for food waste during stirring. The apparent density is calculated by dividing the weight of the carrier by its volume. Specifically, the numerical range of the apparent density can be defined by selecting a lower limit from the group consisting of 300 kg/m$^3$, 350 kg/m$^3$, 400 kg/m$^3$, and 450 kg/m$^3$, and an upper limit from the group consisting of 950 kg/m$^3$, 900 kg/m$^3$, 800 kg/m$^3$, and 750 kg/m$^3$.

[Closed Cell Ratio]

The closed cell ratio of the carrier is preferably 50% or less, more preferably 0 to 40%, even more preferably 0 to 20%, and most preferably 5% to 15%. With a bubble structure within this range of closed cell ratios, microorganisms can more easily penetrate the interior of the carrier and adhere extensively.

Thus, when the carrier has the specified apparent density and closed cell ratio, interconnected pores are formed to carry microorganisms. By impregnating the carrier with a liquid containing microorganisms, the microorganisms can be settled in the pores. Subsequently, stirring multiple carriers with food waste in a treatment apparatus equipped with a stirring function promotes the decomposition of the food waste. Specifically, the numerical range of the closed cell ratio can be defined by selecting a lower limit from the group consisting of 0% and 5%, and an upper limit from the group consisting of 50%, 40%, 20%, and 15%.

The closed cell ratio of the carrier can be measured according to Procedure C of ASTM D2856-70 using an air comparison pycnometer, model 930, manufactured by Toshiba Beckman Co., Ltd.

[Type C Durometer Hardness of Carrier Cross-Section]

The type C durometer hardness (average hardness) of the carrier cross-section is preferably 80 to 100, more preferably 85 to 98, and most preferably 86 to 96. When the cross-section of the carrier has this level of hardness, it exhibits excellent crushing performance for food waste. Specifically, the surface hardness of the carrier can be adjusted to fall within this range by modifying the hardness of the base resin constituting the carrier and by regulating the apparent density of the carrier. From this perspective, the preferred base resin is thermoplastic starch or aliphatic polyester, and more preferably starch-polyester resin or polylactic acid resin. Specifically, the numerical range of the type C durometer hardness can be defined by selecting a lower limit from the group consisting of 80, 85, and 86, and an upper limit from the group consisting of 100, 98, and 96.

The carrier of the present invention is manufactured by extrusion molding using an extruder. For example, the base resin is heated and melted to obtain a molten material, which is extruded and foamed through a die (corresponding to the desired cross-sectional shape) installed at the tip of the extruder (single-screw extruder). A physical blowing agent is injected into the molten material under pressure, while a chemical blowing agent is heated and melted together with the base resin. The extruded and foamed molded product (strand-shaped) is then thoroughly cooled in a water tank and subsequently cut to the desired length using a cutting device (pelletizer), resulting in the columnar carrier.

The carrier is preferably formed into columnar particles by cutting the aforementioned strand. Particularly, when the strand is a foam, cutting the foam reveals a cell structure on the cut surface. This cut surface has an uneven texture. Such uneven areas derived from the cells allow microorganisms to adhere more easily, further enhancing the degradability of the carrier for food waste. From this perspective, the apparent density of the carrier is preferably 350 to 800 $kg/m^3$. The formation of the cell structure contributes to achieving this apparent density.

The blowing agent is not particularly limited and may include, for example, inorganic physical blowing agents such as air, nitrogen, carbon dioxide, argon, helium, oxygen, and neon; aliphatic hydrocarbons such as propane, normal butane, isobutane, normal pentane, isopentane, and normal hexane; alicyclic hydrocarbons such as cyclohexane and cyclopentane; halogenated hydrocarbons such as chlorofluoromethane, trifluoromethane, 1,1-difluoroethane, 1,1,1,2-tetrafluoroethane, methyl chloride, ethyl chloride, and methylene chloride; and organic physical blowing agents such as dimethyl ether, diethyl ether, and methyl ethyl ether. These blowing agents can be used individually or in combinations of two or more. Among these, nitrogen, air, and carbon dioxide are preferred from an environmental perspective, with carbon dioxide being particularly preferred.

The addition amount of the blowing agent is preferably 0.5 to 30 parts by weight per 100 parts by weight of the base resin when using inorganic physical blowing agents, and 5 to 50 parts by weight per 100 parts by weight of the base resin when using organic physical blowing agents.

The cell nucleating agent is not particularly limited and may include inorganic powders such as talc, kaolin, mica, silica, calcium carbonate, barium sulfate, titanium oxide, clay, aluminum oxide, bentonite, and diatomaceous earth. Among these, talc is preferred due to its ease in adjusting cell size.

The addition amount of the cell nucleating agent is preferably 0.1 to 7 parts by weight per 100 parts by weight of the base resin, more preferably 0.2 to 5 parts by weight, and even more preferably 0.3 to 3 parts by weight.

[Examples]

The present invention will be described in detail below with reference to examples. However, the invention is not limited to the examples described herein.

Carriers with a star-shaped cross-section were produced for Examples 1 to 7 and Comparative Examples 1 and 2 using the compositions shown in Table 1.

[Table 1]

|  |  | Example 1 | Example 2 | Example 3 | Example 4 | Example 5 | Example 6 | Example 7 | Comparative Example 1 | Comparative Example 2 |
|---|---|---|---|---|---|---|---|---|---|---|
| PLA | parts by weight | 100 | 100 | 100 | - | - | - | - | - | - |
| Starch polyester | parts by weight | - | - | - | 100 | 100 | 100 | - | - | - |
| PBS | parts by weight | - | - | - | - | - | - | 100 | - | - |
| PP | parts by weight | - | - | - | - | - | - | - | 100 | - |
| PVA | parts by weight | - | - | - | - | - | - | - |  | 100 |
| Cell nucleating agent (talc) | % by weight | 1 | 1 | 1 | 1 | 1 | 1 | 1 | - | 1 |
| Physical blowing agent (CO2) | % by weight | 1 | 0.4 | 0.5 | 1 | 1.8 | 2.1 | 2.4 | - | 0.6 |
| Chemical blowing agent | % by weight | - | - | - | - | - | - | - | 5 | 0 |

The details of each component in Table 1 are as follows:

(1) PLA (Polylactic Acid): "LX975" (amorphous PLA, D-isomer content 12%, plant-based content 99.9%, manufactured by Total Corbion)

(2) Starch Polyester Resin: "Mater-Bi" (plant-based content 100%, manufactured by Novamont) ※
Starch polyester resin is a resin composed of thermoplastic starch and PBAT (plant-based content 100%). The amount of thermoplastic starch is approximately 30% by weight when the total of thermoplastic starch and PBAT is set to 100% by weight.

(3) PBS (Polybutylene Succinate): "FZ91PB" (plant-based content 48.5%, manufactured by Mitsubishi Chemical)

※ The plant-based content of PLA and PBS was referenced from the BiomassPla PL list by the Japan Bioplastics Association.

(4) PP (Polypropylene): "J-750HP" (b-PP, petroleum-based thermoplastic resin, manufactured by Prime Polymer)
(5) PVA (Polyvinyl Alcohol): "Poval C-500T" (density 1.3 g/cm$^3$, manufactured by Kuraray)
(6) Cell Nucleating Agent: Talc ("Hi-Filler 5000PJ," manufactured by Matsumura Sangyo)
(7) Physical Blowing Agent: Carbon dioxide (CO2, physical blowing agent)
(8) Chemical Blowing Agent: "PO217K" (citric acid-bicarbonate-based, manufactured by Dainichiseika)

PLA, starch polyester resin, PBS, and PVA are biodegradable resins (biodegradability of 60% or higher), while PP is non-biodegradable resin (biodegradability of less than 60%).

The carriers for Examples 1 to 7 and Comparative Examples 1 and 2 were produced by heating and melting the base resin and the cell nucleating agent to obtain a molten material, which was then extruded and foamed through a star-shaped die installed at the tip of an extruder (Machine I: φ50 mm, L/D = 50, single-screw extruder). The extruded and foamed molded product (strand) was then sufficiently cooled in a 4-meter water tank and cut using a pelletizer (large fan cutter manufactured by Hoshi Plastic) to obtain the carriers. The physical blowing agent was injected into the molten material, while the chemical blowing agent was heated and melted together with the base resin. The cell nucleating agent, physical blowing agent, and chemical blowing agent shown in Table 1 are the amounts added per 100% by weight of the base resin consisting of each resin.

The carriers of Examples 1 to 7 and Comparative Examples 1 and 2 were evaluated for apparent density, closed cell ratio, average particle size, arithmetic average height (Sa), weight loss rate, and food waste degradability. The results are shown in Table 2.

[Table 2]

| | | Example 1 | Example 2 | Example 3 | Example 4 | Example 5 | Example 6 | Example 7 | Comparative Example 1 | Comparative Example 2 |
|---|---|---|---|---|---|---|---|---|---|---|
| Apparent density | kg/m³ | 490 | 470 | 380 | 540 | 640 | 930 | 700 | 350 | 770 |
| Closed cell ratio | | 0 | 98 | 23 | 8 | 10 | 20 | 14 | 2 | 1 |
| Average particle size | mm | 11.1 | 11.1 | 11 | 10.2 | 8.9 | 8.9 | 10 | 11.9 | 11.9 |
| Volume per carrier | cm3 | 0.70 | 0.73 | 0.71 | 0.56 | 0.45 | 0.31 | 0.62 | 0.72 | 0.63 |
| Type C durometer hardness | C | 95 | 94 | 88 | 83 | 88 | 98 | 93 | 86 | 81 |
| Number of micropores on the surface | count/100 $\mu m^2$ | 0 | 0 | 0 | 41 | 55 | 50 | 0 | 0 | 0 |
| Sa | μm | 3.6 | 2.3 | 2.9 | 2.6 | 3.1 | 3.6 | 2.5 | 2.1 | 7.5 |
| Weight loss rate after 1 hour | % | 62 | 47 | 46 | 52 | 55 | 59 | 50 | 44 | 8 |
| Food waste degradability | ◎・○・× | ○(good) | ○(good) | ○(good) | ◎(excellent) | ◎(excellent) | ◎(excellent) | ○(good) | ○(good) | ×(poor) |

[Biodegradability]

The biodegradability of the carriers can be measured based on the standard method of JIS K6953 (corresponding to JIS K6953-1). The biodegradability of the carriers is calculated as: (Total $CO_2$ emission during 90 days in a compost environment) / (Theoretical $CO_2$ emission calculated from the composition formula) $\times 100$. Using the above method, the biodegradability of the carriers in Examples 1 to 7 and Comparative Example 2 was 60% or higher, while the biodegradability of the carrier in Comparative Example 1 was 0%.

[Apparent Density]

The apparent density was calculated by dividing the weight of the carrier by the volume determined using the water displacement method, in which the carrier is submerged in a graduated container filled with water, and the rise in water level is measured.

[Closed Cell Ratio]

The closed cell ratio was calculated following Procedure C described in ASTM D2856-70, using an air comparison pycnometer (model 930, manufactured by Toshiba Beckman Co., Ltd.). The true volume Vx of the carrier was measured, and the closed cell ratio S (%) was determined using the following formula (1). The average value for N=3 was used:

$$S\,(\%)＝(Vx－W/\rho)\times100/\,(VA－W/\rho)\cdot\cdot\cdot(1)$$

Where:

Vx: True volume of the sample ($cm^3$) measured by the method. This corresponds to the sum of the volume of the resin constituting the carrier sample and the total volume of the closed cells within the sample.
VA: Apparent volume of the sample ($cm^3$), calculated by dividing the weight of the sample by its apparent density.
W: Total weight of the sample (g).
$\rho$: Density of the resin constituting the carrier ($g/cm^3$).

[Average Particle Size]

The average particle size was determined by measuring the maximum height of individual columnar carriers using a caliper or similar tool and calculating the average value (N = 3).

[Arithmetic Average Height (Sa)]

The arithmetic average height (Sa) was determined using analysis software (VHX-H5M) from a 3D image of the side surface of the carrier captured with a digital microscope (VHX-7000, non-contact type). The 3D image was corrected for flatness using an L-filter with a cutoff value of 0.25 mm.

[Weight Loss Rate]

Each carrier (3 mL) and a test specimen (Neoma Foam, manufactured by Asahi Kasei) with a 10 mm square cross-section and a length of 60 mm were placed in a sample bottle with a diameter of $\varphi40$ mm and a height of 120 mm containing 35 mL of water and a stirrer. A stirring test was conducted using a magnetic stirrer at a stirring speed of 1,000 rpm. Every hour, the test specimen was removed from the sample bottle, thoroughly dried, and weighed. The weight loss rate W (%) was calculated using the following formula (2) for evaluation:

$$W\,(\%) = (wb - wa) \times 100 / wa \quad (2)$$

In formula (2), wa and wb are as follows:

wa: Weight of the test specimen before the test (g)
wb: Weight of the test specimen 1 hour after the start of the test (g)

The number of tests was set to n=3.

[Food Waste Decomposition Test]

The food waste decomposition test was conducted using the food waste processor SINKPIA GJ-20 (manufactured by SINKPIA Japan). A carrier (20 L) in which microorganisms had been established and quartered cabbage (2.5 kg) were placed into the processor. After stirring for two days, the weight of the cabbage residue in the processor was measured, and the decomposition rate C (%) was calculated using the following formula (3) to evaluate the decomposition performance:

$$C(\%)=(A-B)\times100/(A)\cdots(3)$$

In formula (3), A and B are defined as follows:

A: Weight of the cabbage before the test (g)
B: Weight of the cabbage residue in the processor 48 hours after the start of the test (g)

The evaluation criteria for food waste decomposition performance are as follows:

If the decomposition rate of the cabbage, calculated using formula (3), is 80% or higher after two days from the start of the test and no core parts of the cabbage remain: ◎ (excellent)
If the decomposition rate of the cabbage, calculated using formula (3), is 80% or higher after two days from the start of the test, but some core parts of the cabbage remain: ∘ (good)
If the decomposition rate of the cabbage, calculated using formula (3), is less than 80% after two days from the start of the test: × (poor)

[Type C Durometer Hardness of Carrier Cross-Section]

The type C durometer hardness of the carrier cross-section refers to the hardness measured based on JIS K7312 (1996) using a type C durometer (Asker C hardness tester). The measurement specifically targets the cell membrane portion of the carrier cross-section.

[Surface Condition of the Side Portion of the Carrier]

An electron microscope image (magnified 10,000 times) of the side portion of the columnar carrier was captured. In the obtained image, the number of pores with a maximum diameter of 0.3 to 3 $\mu$m within an area of 100 $\mu$m$^2$ was measured. In Examples 4 to 6, where starch polyester was used as the base resin, the presence of such pores was confirmed. In Example 4, 42 pores per 100 $\mu$m$^2$ were observed, in Example 5, 58 pores per 100 $\mu$m$^2$ were observed, and in Example 6, 42 pores per 100 $\mu$m$^2$ were observed.
In Examples 1 to 7, the carriers were biodegradable as their biodegradability was 60% or higher. On the other hand, in Comparative Example 1, the biodegradability was 0%, indicating poor biodegradability of the carrier. Additionally, in Comparative Example 2, neither thermoplastic starch nor aliphatic polyester was included in the base resin, causing the carrier to dissolve due to the moisture in the food waste or water generated during its decomposition. As a result, the carrier failed to exhibit effective food waste decomposition performance.

**Claims**

1. A microbial carrier for food waste treatment,
   wherein the base resin constituting the microbial carrier comprises thermoplastic starch and/or aliphatic polyester, the microbial carrier has a biodegradability of 60% or higher in a biodegradability test based on JIS K6953, and the microbial carrier has a columnar shape.

2. The microbial carrier for food waste treatment according to claim 1,
   wherein the arithmetic average height (Sa) of surface irregularities on the surface of the microbial carrier is 2.4 $\mu$m or more.

3. The microbial carrier for food waste treatment according to claim 1 or 2,
   wherein the apparent density of the microbial carrier is 400 kg/m$^3$ or more and 800 kg/m$^3$ or less.

4. The microbial carrier for food waste treatment according to any one of claims 1 to 3,
   wherein the cross-sectional shape of the microbial carrier, when cut perpendicularly to the height direction, is polygonal.

5. The microbial carrier for food waste treatment according to any one of claims 1 to 4,
   wherein the plant-based content of the thermoplastic starch or aliphatic polyester, as determined based on ASTM D6866, is 40% or more.

FIG.1

FIG.2

FIG.3

FIG.4

FIG.5

FIG.6

X

FIG.7

FIG.8

## INTERNATIONAL SEARCH REPORT

| International application No. |
|---|
| **PCT/JP2023/030990** |

### A. CLASSIFICATION OF SUBJECT MATTER

*C08J 9/04*(2006.01)i; *C12N 1/00*(2006.01)i
FI:  C08J9/04 101; C12N1/00 P; C08J9/04 CFD

According to International Patent Classification (IPC) or to both national classification and IPC

### B. FIELDS SEARCHED

Minimum documentation searched (classification system followed by classification symbols)

C08J9/00-9/42; C12N1/00-7/08; B09B1/00-5/00; B09C1/00-1/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | US 2020/0189946 A1 (NANO AND ADVANCED MATERIALS INSTITUTE LIMITED) 18 June 2020 (2020-06-18)<br>entire text | 1-5 |
| A | JP 4-504380 A (ALLIED SIGNAL INC) 06 August 1992 (1992-08-06)<br>entire text | 1-5 |
| A | WO 2013/149662 A1 (HERA S.P.A.) 10 October 2013 (2013-10-10)<br>entire text | 1-5 |
| A | WO 2013/051648 A1 (IGA BIO RESEARCH CO., LTD.) 11 April 2013 (2013-04-11)<br>entire text | 1-5 |
| A | JP 2015-503327 A (XYLECO, INC) 02 February 2015 (2015-02-02)<br>entire text | 1-5 |
| A | WO 2022/085725 A1 (DAICEL MIRAIZU LTD.) 28 April 2022 (2022-04-28)<br>entire text | 1-5 |

✓ Further documents are listed in the continuation of Box C.     ✓ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **08 November 2023** | **21 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2023/030990**

**C.  DOCUMENTS CONSIDERED TO BE RELEVANT**

| Category* | Citation of document, with indication, where appropriate, of the relevant passages | Relevant to claim No. |
|---|---|---|
| A | JP 2002-293982 A (KANEBO LTD) 09 October 2002 (2002-10-09)<br>entire text | 1-5 |
| A | JP 11-290825 A (KANEBO LTD) 26 October 1999 (1999-10-26)<br>entire text | 1-5 |

Form PCT/ISA/210 (second sheet) (January 2015)

## INTERNATIONAL SEARCH REPORT
### Information on patent family members

International application No.

**PCT/JP2023/030990**

| Patent document cited in search report | | | Publication date (day/month/year) | Patent family member(s) | | | Publication date (day/month/year) |
|---|---|---|---|---|---|---|---|
| US | 2020/0189946 | A1 | 18 June 2020 | WO | 2018/210242 | A1 | |
| | | | | entire text | | | |
| | | | | CN | 110650924 | A | |
| JP | 4-504380 | A | 06 August 1992 | US | 4983299 | A | |
| | | | | entire text | | | |
| | | | | US | 5543052 | A | |
| | | | | WO | 1990/011970 | A1 | |
| | | | | EP | 467969 | A1 | |
| | | | | KR | 10-1992-0701053 | A | |
| WO | 2013/149662 | A1 | 10 October 2013 | (Family: none) | | | |
| WO | 2013/051648 | A1 | 11 April 2013 | EP | 2764931 | A1 | |
| | | | | entire text | | | |
| | | | | KR | 10-2014-0074967 | A | |
| | | | | CN | 103987467 | A | |
| | | | | TW | 201323607 | A | |
| JP | 2015-503327 | A | 02 February 2015 | US | 2014/0011258 | A1 | |
| | | | | entire text | | | |
| | | | | US | 2016/0076014 | A1 | |
| | | | | US | 2017/0022530 | A1 | |
| | | | | US | 2017/0268029 | A1 | |
| | | | | US | 2018/0223320 | A1 | |
| | | | | US | 2019/0233863 | A1 | |
| | | | | WO | 2013/096699 | A1 | |
| | | | | WO | 2013/096698 | A1 | |
| | | | | EP | 2794894 | A1 | |
| | | | | EP | 2794895 | A1 | |
| | | | | CN | 103998614 | A | |
| | | | | CN | 104039972 | A | |
| | | | | KR | 10-2014-0108533 | A | |
| | | | | KR | 10-2014-0111662 | A | |
| | | | | JP | 2015-503326 | A | |
| | | | | JP | 2017-205115 | A | |
| | | | | JP | 2018-7665 | A | |
| WO | 2022/085725 | A1 | 28 April 2022 | CN | 116096513 | A | |
| | | | | TW | 202222985 | A | |
| JP | 2002-293982 | A | 09 October 2002 | (Family: none) | | | |
| JP | 11-290825 | A | 26 October 1999 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003055562 A
- JP 6869307 B